# EUROPEAN PATENT SPECIFICATION

(11) **EP 3 215 036 B1**
(45) Date of publication and mention of the grant of the patent: **27.12.2023**
(21) Application number: 15857822.9
(22) Date of filing: 06.11.2015
(51) Int. Cl.: A61B 17/70, A61F 2/44

(54) **INTERCONNECTED LOCKING PLATES FOR ADJACENT SPINAL VERTEBRAL BODIES**
VERBUNDENE VERSCHLUSSPLATTEN FÜR ANGRENZENDE RÜCKENWIRBELKÖRPER
PLAQUES DE VERROUILLAGE INTERCONNECTÉES POUR DES CORPS VERTÉBRAUX ADJACENTS

(30) Priority: 06.11.2014 US 201414534884
(43) Date of publication of application: 13.09.2017
(73) Proprietor: VGI Medical, LLC, Largo, FL 33777 (US)
(72) Inventor: VESTGAARDEN, Tov, Inge, Madeira Beach, FL 33708 (US)
(74) Representative: Cullinane, Marietta Bettina
(86) International application number: PCT/US2015/059462
(87) International publication number: WO 2016/073849

(56) References cited:
- US-A1- 2002 161 441
- US-A1- 2008 161 926
- US-A1- 2011 166 600
- US-A1- 2012 239 089
- US-A1- 2014 324 103

## Description

### BACKGROUND OF THE INVENTION

### FIELD OF THE INVENTION

1. This invention relates to a device for insertion into the gap between two adjacent vertebral bodies.

### 2. BRIEF DESCRIPTION OF THE RELATED ART

In disc herniation, a spinal disc bulges from between two vertebral bodies and impinges on adjacent nerves, causing pain. In some cases, non-operative procedures such as bed rest, medication, lifestyle modifications, exercise, physical therapy, chiropractic care and steroid injections may suffice. However, in other cases, surgical intervention may be necessary. In cases where surgical intervention is prescribed, spinal vertebral body fusion may be desirable, i.e., the spine may have deteriorated so much that adjacent vertebral bodies must be fused together.

Spinal fixation is the current standard of care for surgically treating disc herniation in patients who have chronic pain and who have, or are likely to develop, associated spinal instability. Spinal fixation procedures are intended to relieve impingement on nerves by removing the portion of the disc or bone, or both, responsible for compressing the neural structures and destabilizing the spine.

The prior art teaches that excised disc or bone must be replaced with one or more intervertebral implants, or spacers, placed between adjacent vertebral bodies. These implants stabilize the adjacent vertebral bodies relative to one another so that the two vertebral bodies can fuse together.

US 2002/161 441 A1 describes an implant. In particular this document discloses a vertebral bone prosthetic device comprising an interior body, provided with an outer surface and a first coupling element thereon, and an exterior hollow body with a bore therein extending along a central axis, with the interior body being configured and dimensioned to be slidably received by the exterior body along the central axis, and provided with an interior surface and an exterior surface, the interior surface having a groove. The device also comprises a fixation ring having inner and outer surfaces, with the inner surface having a second coupling element thereon. The fixation ring is configured and dimensioned to be received within the groove of the exterior body for rotational movement about the central axis of the exterior body. Rotation of the fixation ring results in engagement of the first and second coupling elements to thereby prevent relative sliding movement between the interior and exterior bodies. Also, the fixation ring is rotatable between first second positions, the first position disengaging the coupling elements and allowing relative sliding movement and the second position engaging the coupling elements and blocking relative sliding movement.

US 2008/161 926 A1 discloses implants with inner and outer members positioned in a telescoping arrangement. The members may include helical supports to selectively adjust the height of the implant. The helical supports offer a large contact surface to prevent inadvertent reduction of the length due to application of a compressive force. In some embodiments, the helical support members may be shaped to facilitate movement of the members to increase the height. The shape may further prevent movement that would decrease the height. Some embodiments may also prevent relative rotation of the members. In use, the inner and outer members are moved apart to adjust the height of the implant to space apart the vertebral members. The members may further be constructed to prevent the members from moving together after insertion between the vertebral members.

The prior art considered as a whole at the time the present invention was made does not include any discussion concerning whether or not spinal fixation can be accomplished in the absence of a spinal fusion implant. It necessarily follows that it was not obvious to those of ordinary skill in the art at the time the present invention was made that spinal fixation could be accomplished in the absence of spinal fusion implants.

### SUMMARY OF THE INVENTION

The present invention relates to a device for insertion into a gap between two adjacent vertebral bodies, as presented in claim 1. Preferred embodiments are set forth in the dependent claims. The long-standing but heretofore unfulfilled need for an improved spinal fusion implant is now met by a new, useful, and non-obvious invention.
US 2002/161 441 A1 describes such an implant. In particular this document discloses a vertebral bone prosthetic device comprising an interior body, provided with an outer surface and a first coupling element thereon, and an exterior hollow body with a bore therein extending along a central axis, with the interior body being configured and dimensioned to be slidably received by the exterior body along the central axis, and provided with an interior surface and an exterior surface, the interior surface having a groove. The device also comprises a fixation ring having inner and outer surfaces, with the inner surface having a second coupling element thereon. The fixation ring is configured and dimensioned to be received within the groove of the exterior body for rotational movement about the central axis of the exterior body. Rotation of the fixation ring results in engagement of the first and second coupling elements to thereby prevent relative sliding movement between the interior and exterior bodies. Also, the fixation ring is rotatable between first second positions, the first position disengaging the coupling elements and allowing relative sliding movement and the second position engaging the coupling elements and blocking relative sliding movement.
US 2008/161 926 A1 discloses implants with inner and outer members positioned in a telescoping arrangement. The members may include helical supports to selectively adjust the height of the implant. The helical supports offer a large contact surface to prevent inadvertent reduction of the length due to application of a compressive force. In some embodiments, the helical support members may be shaped to facilitate movement of the members to increase the height. The shape may further prevent movement that would decrease the height. Some embodiments may also prevent relative rotation of the members. In use, the inner and outer members are moved apart to adjust the height of the implant to space apart the vertebral members. The members may further be constructed to prevent the members from moving together after insertion between the vertebral members.

The novel structure does not include a spinal fusion implant having a main body having a predetermined length, width, and thickness as disclosed in all prior art literature relating to spinal fusion implants.

However, the novel structure stops motion between adjacent vertebral bodies, thereby allowing fusion to occur. Conventional fusion accelerants, such as bone, are added to the empty space conventionally occupied by a spinal fusion implant main body in order to promote fusion.

The novel main body-less spinal fusion implant is inserted into a gap between adjacent, spaced apart vertebral bodies in a spinal joint, said gap created by surgical removal of a disc.

The present invention relates to a device as defined in claim 1. Preferred embodiments are described in the dependent claims. The novel spinal fusion implant includes an elongate interconnecting member that may have an adjustable length, a distal retention plate rotatably secured to a distal end of the interconnecting member, and a proximal retention
plate secured to a proximal end of the interconnecting member.

The distal retention plate has an unrotated position of repose and an infinite plurality of rotated positions relative to said position of repose. Similarly, the proximal retention plate may have an unrotated position of repose and an infinite plurality of rotated positions relative to the position of repose.

When the distal and proximal retention plates are in their respective positions of repose, they are rotationally aligned with one another.

The proximal retention plate may be fixedly secured into its operable position so that it does not rotate relative to the interconnecting member but can be rotated conjointly with the interconnecting member.

The distal retention plate is in its position of repose when it is inserted into the surgically-created gap between adjacent vertebral bodies and is in a rotated position after the distal retention plate has cleared the distal surface of the spinal vertebral bodies, i.e., when the distal retention plate has passed through and is not positioned in said gap.

The distal retention plate when in the second position after said insertion abuts the superior and inferior vertebral bodies on the distal side of the spine and therefore prevents distal-to-proximal travel of the distal retention plate and thus prevents retraction of the interconnecting member from the gap and cooperates with the rotated proximal retention plate to hold adjacent vertebral bodies in a stable relationship to one another.

After the distal retention plate has cleared the distal spinal vertebral bodies and is rotated to prevent its re-entry into the gap, the interconnecting member is shortened to cause the proximal and distal retention plates to converge toward one another, thereby tightly sandwiching the adjacent vertebral bodies between them and holding said adjacent vertebral bodies against movement.

The rotation of the distal retention plate is preferably a ninety degree (90°) rotation relative to its position of repose, but the distal retention plate can still perform its function when rotated less than ninety degrees (90°) and such reduced angles of rotation are within the scope of this invention.

Both retention plates may have a roughened inboard surface to enhance their respective grips on their respective vertebral bodies.

Both retention plates may also have at least one protrusion formed on an inboard surface thereof to enhance their respective grips on their respective vertebral bodies.

The maximum length of the adjustable length interconnecting member is sufficient to span the proximal-to-distal extent of the gap and to allow the proximal and distal retention plates to be positioned outside the gap. The minimum length of the adjustable length interconnecting member is sufficient to enable the distal and proximal retention plates to tightly grip the adjacent vertebral bodies in sandwiched relation between them.

The proximal retention plate may be in its unrotated position of repose, rotationally aligned with the distal retention plate during distal retention plate insertion, or it may be rotated into its deployed configuration prior to insertion of the distal retention plate because the proximal retention plate does not enter into the gap.

The length of the interconnecting member is fixed or adjustable by any suitable mechanical means. The retention plates converge toward one another when the length of the interconnecting member is shortened, thereby holding the adjacent vertebral bodies in a stable relationship to one another as aforesaid.

The suitable mechanical means includes providing the interconnecting member in telescoping form.

According to the invention, the device does not require incisions on both sides of the spine, thereby obtaining an important object of the invention, i.e., providing a spinal fusion device that does not require incisions on both sides of a spine.

Another important object is to provide a spinal fusion device having no main body as in all prior art spinal fusion devices.

A more specific object is to provide a spinal fusion device that is inserted from a proximal side of a spine and which has a distal retention plate mounted on the distal end of an adjustable length interlocking member where the distal retention plate is aligned with a gap formed between adjacent vertebral bodies and inserted through said gap until the distal retention plate clears the distal edge of the adjacent vertebral bodies.

A closely related object is to provide a device that is inserted from a proximal side of a spine and which has a proximal retention plate mounted on the proximal end of an adjustable length interlocking member where the proximal retention plate may be aligned with the gap formed between adjacent vertebral bodies but which is not inserted into said gap.

Another closely related object is to provide a tool where distal and proximal retention plates are respectively mounted to distal and proximal ends of an adjustable length interconnecting member and where the distal retention plate is in a non-rotated position during insertion and in a rotated, deployed configuration after insertion.

These and other important objects, advantages, and features of the invention will become clear as this disclosure proceeds.

The invention accordingly comprises the features of construction, combination of elements, and arrangement of parts that will be exemplified in the disclosure set forth hereinafter and the scope of the invention will be indicated in the claims.

### BRIEF DESCRIPTION OF THE DRAWINGS

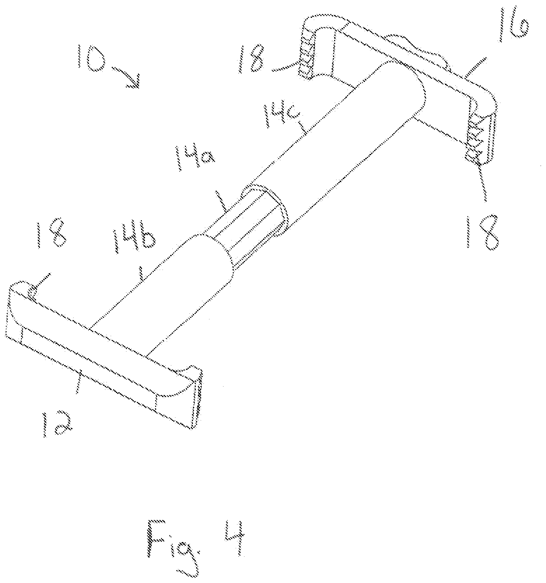

The invention is illustrated in figures 4, 5 and 6. The surgical methods are not according to the invention and are present for illustration purposes only.

For a fuller understanding of the nature and objects of the invention, reference should be made to the following detailed disclosure, taken in connection with the accompanying drawings, in which:
Fig. 1 is a perspective view of a first embodiment of a device not according to the invention,
Fig. 2 is a top plan view thereof,
Fig. 3 is a perspective view of a second not according to the invention,
Fig. 4 is a perspective view of a third embodiment,
Fig. 5 is a perspective view of a fourth embodiment, and
Fig. 6 is a perspective view of a fifth embodiment.

### DETAILED DESCRIPTION OF THE PREFERRED EMBODIMENT

A spine includes a plurality of vertebral bodies separated by discs. A spine may deteriorate so much that adjacent vertebral bodies must be fused together. The novel spinal fusion implant is disposed between contiguous vertebral bodies to immobilize the affected segment of the spine and facilitate fusion between said adjacent vertebral bodies.

Prior art spinal fusion devices include a main body that substantially fills the gap between adjacent vertebral bodies. However, it has been discovered by the present inventor that fusion can occur in the absence of a main body. With a lower mass in the disc space, there is an increased chance, but no guarantee, of subsidence. Any structure with bleeding bone and micro motion will grow together. In this case the bleeding bone will grow to the distal and proximal plates where they attach to the vertebral bodies or directly between adjacent vertical bodies. If a surgeon adds autograft, allograft, or biologics to the disc space, this will be the traditional method of fusion.

The spine is prepared by removing some or all of the disc that resides in the space where the novel spinal fusion implant is to be inserted. The disc space is prepared with a rongeur or other surgical instrument, not depicted.

The novel structure effectively stabilizes the joint but permits the occurrence of "micro-motion" between the opposing vertebral bodies, which is important for successful bone fusion.

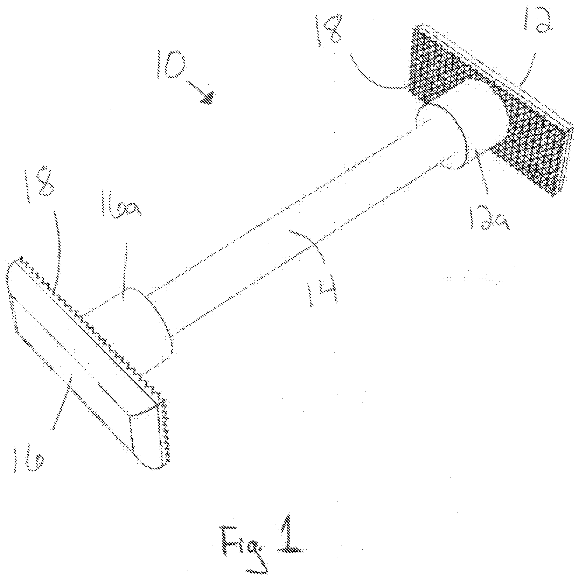

Figure 1 shows an embodiment which is not according to the invention. Referring now to Figs. 1-3, tool 10 includes distal retention plate 12 which is attached to the distal end of elongate interconnecting member 14. Proximal retention plate 16 is attached to the proximal end of said elongate interconnecting member 14. Proximal retention plate 16 may be mounted for relative rotation with respect to interconnecting member 14 or it may be fixedly secured thereto or integrally formed therewith for conjoint rotation therewith.

Boss 12a may be formed integrally with distal retention plate 12 and boss 16a may be formed integrally with proximal retention plate 16, said bosses receiving the opposite ends of interconnecting member 14 to enhance the structural integrity of the novel structure. There is no relative rotation and no longitudinal displacement between the bosses and said opposite ends.

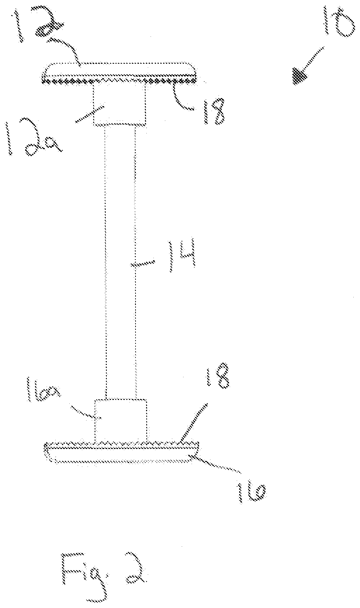

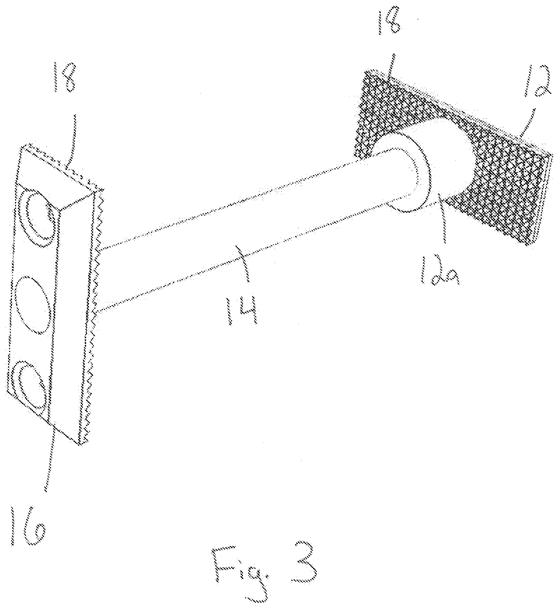

In the embodiments of Figs. 1-3, rotation of proximal retention plate 16 by a surgeon effects conjoint rotation of distal retention plate 12. Said plates may be in rotational alignment with one another in a first embodiment as depicted in Figs. 1 and 2, or they may be rotated ninety degrees (90°) with respect to one another in a second embodiment as depicted in Fig. 3. Other angular orientations between the retention plates.

The first embodiment not forming part of the invention as illustrated in Figure 1 and 2 is a non-telescoping embodiment. All other embodiments have telescopically interconnected parts. The first embodiment could also be modified so that the distal and proximal plates could be telescopically interconnected to one another.

As shown in Fig. 3, proximal retention plate 16 has at least two (2) openings that receive screws so that plate 16 may be secured to the vertebral bodies.

Each retention plate preferably has a roughened inboard surface as at 18. The inboard surface is the surface that abuts the patient's body. At least one protuberance may also be formed in the respective inboard surfaces of distal and proximal retention plates 12 and 16, respectively. Such protuberances would perform the same gripping function as the aforesaid roughened surfaces. The use of only one (1) protrusion is considered the equivalent of a roughened surface.

Fig. 4 depicts a third embodiment. Retention plates 12 and 16 in this embodiment have a square bracket ( [ ) shape with rounded corners and the retention plates 12, 16 contact the patient's body only at the inboard-projecting opposite ends of such retention plates. The body-contacting opposite ends have roughened surfaces 18 similar to the roughened surfaces of the first embodiment.

Elongate interconnecting member 14 in this embodiment has three parts, i.e., elongate base 14a having an octagonal transverse cross-section, distal part or sleeve 14b which is formed integrally with or fixedly secured to distal retention plate 12 for conjoint rotation therewith, and proximal part or sleeve 14c which is formed integrally with or fixedly secured to proximal retention plate 16 for conjoint rotation therewith.

Parts 14b and14c are provided with octagonal lumens that telescopically mate with elongate base 14a. A surgeon may insert distal retention plate 12 through the disc space until said distal retention plate clears the vertebrae while holding proximal retention plate 16 in the position depicted in Fig. 4. Rotation of proximal retention plate 16 then effects conjoint rotation of distal retention plate 12 just as in the first three embodiments. The difference is that said retention plates of this second embodiment are telescopically interconnected so that tool 10 can be used with patients of varying sizes. Thus it is understood that the transverse cross-section of base 14a and the mating lumens of parts 14b and 14c could be of any non-round cross-section.

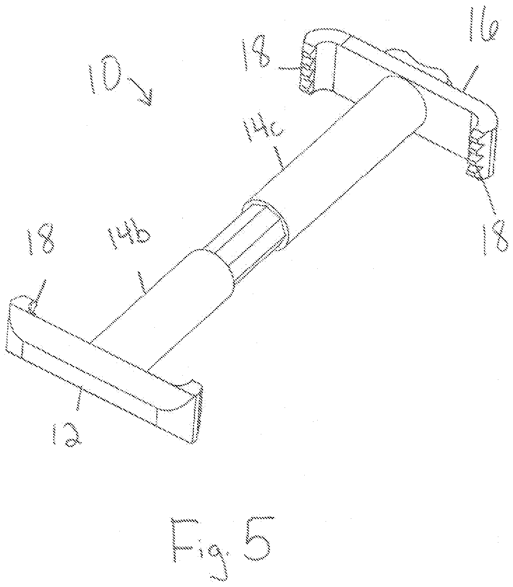

A fourth embodiment is depicted in Fig. 5. This embodiment eliminates base member 14a. Distal and proximal parts 14b, 14c telescopically engage one another. More particularly 14c has an internal lumen designed to accept the cross-sectional shape of 14b.

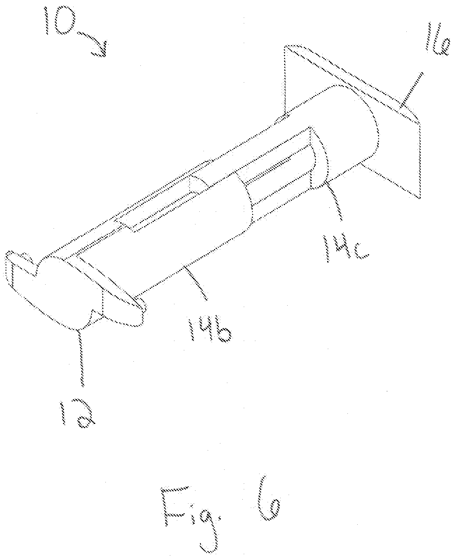

A fifth embodiment is depicted in Fig. 6. This embodiment also eliminates base member 14a. Distal and proximal parts 14b, 14c telescopically engage one another. More particularly, each part 14b, 14c has two diametrically oposed arms and two diametrically opposed slots slots so that the arms of distal part 14b slidingly engage the slots of proximal part 14c and the arms of proximal part 14c slidingly engage the slots of distal part 14b.

Distal retention plate 12 in Fig. 6 has a different structure than proximal retention plate 16 to allow plate 12 to cut through surrounding soft tissue.

Misalignment (at least some rotation) of distal retention plate 12 from its non-rotated position limits motion in a multi-directional joint. More particularly, the shape of the main body in the incorporated disclosure limits motion in flexion/extension, while distal retention plate 12 in cooperation with proximal retention plate 16 limits lateral bending. Accordingly, this main body-less embodiment does not limit motion in flexion/extension.

Thus it is understood that the spine is locked in sandwiched relation between proximal retention plate 16 on the proximal side and distal retention plate 12 on the distal side even though only one incision has been made, said incision being on said proximal side, thereby distinguishing the invention from prior art tools and methods that require two (2) incisions, i.e., incisions on both the proximal and the distal side of the spine.

Spinal fusion implant 10 is inserted into a disc space using a lateral approach. The lateral approach is preferred because it is familiar to spine surgeons, and also minimizes the possibility of damage to the spinal cord during insertion of the tool.

Although tool 10 has been disclosed in the context of fusing an intervertebral joint, it may also be used to stabilize and fuse any joint having an anatomy similar to an intervertebral joint, i.e., a pair of opposing bony surfaces defining a gap therebetween. By way of example and not limitation, the novel tool may be used in small joints as in the finger, toe, etc.

It will thus be seen that the objects set forth above, and those made apparent from the foregoing disclosure, are efficiently attained. Since certain changes may be made in the above construction without departing from the scope of the invention, it is intended that all matters contained in the foregoing disclosure or shown in the accompanying drawings shall be interpreted as illustrative and not in a limiting sense.

It is also to be understood that the following claims are intended to cover all of the generic and specific features of the invention herein disclosed, and all statements of the scope of the invention that, as a matter of language, might be said to fall therebetween.

## Claims

1. A device (10) for insertion into the gap between two adjacent vertebral bodies, comprising:
an elongate interconnecting member (14)
a distal retention plate (12) comprising a distal outboard surface and a proximal inboard surface, said distal retention plate (12) having a distal part (14b) of the interconnecting member (14) secured in normal relation thereto and extending proximally therefrom;
a proximal retention plate (16) comprising a distal inboard surface and a proximal outboard surface, said proximal retention plate (16) having a proximal part (14c) of the interconnecting member (14) secured in normal relation thereto and extending distally therefrom;
said distal part (14b) of the interconnecting member (14) of said distal retention plate (12) being telescopically mounted to said proximal part (14c) of the interconnecting member (14) of said proximal retention plate (16) to form the interconnecting member (14) for selectively moving said distal retention plate (12) away from, or toward, said proximal retention plate (16), and for selectively rotating said distal retention plate (12) relative to said proximal retention plate (16) and for selectively rotating said proximal retention plate (16) relative to said distal retention plate (12);
wherein said device (10) is adapted for being inserted into the gap between two adjacent vertebral bodies and the interconnecting member has a maximum length sufficient to span the proximal-to-distal extent of the gap and to allow the proximal and distal retention plates to be positioned outside of the gap and a minimum length sufficient to enable the distal and proximal retention plates to tightly grip the adjacent bodies in sandwiched relation between them..

2. The device (10) of claim 1, wherein said distal retention plate (12) and said proximal retention plate (16) are sufficiently large so as to span a distance between said adjacent vertebral bodies when rotated after insertion.

3. The device (10) of claim 2, wherein said distal part (14b) of the interconnecting member (14) of said distal retention plate (12) and said proximal part (14c) of the interconnecting member (14) of said proximal retention plate (16) are secured to one another in telescoping and non-rotating relation.

4. The device (10) of claim 3, wherein said distal part (14b) of the interconnecting member (14)of said distal retention plate (12) and said proximal part (14c) of the interconnecting member (14)of said proximal retention plate (16) comprise a non-round transverse cross-section; and
said distal part (14b) of the interconnecting member (14)of said distal retention plate (12) comprises a non-round lumen that telescopically mates with a non-round transverse cross-section of said proximal part (14c) of the interconnecting member (14)of said proximal retention plate (16);
whereby rotation of said proximal retention plate (16) effects conjoint rotation of said distal retention plate (12).

5. The device (10) of claim 3, wherein said distal part (14b) of the interconnecting member (14) of said distal retention plate (12) and said proximal part (14c) of the interconnecting member (14) of said proximal retention plate (16) comprise a non-round transverse cross-section; and
said proximal part (14c) of the interconnecting member (14) of said proximal retention plate (16) comprises a sleeve having a non-round lumen, said sleeve being configured to telescopically mate with a non-round transverse cross-section of said distal part (14b) of the interconnecting member (14) of said distal retention plate (12);
whereby rotation of said proximal retention plate (16) effects conjoint rotation of said distal retention plate (12).

6. The device (10) of claim 2, wherein-said distal part (14b) of the interconnecting member (14) of said distal retention plate (12) and said proximal part (14c) of the interconnecting member (14) of said proximal retention plate (16) have a length sufficient to enable said proximal retention plate (16) and said distal retention plate (12) to extend beyond the adjacent vertebral bodies.

7. The device (10) of claim 2, wherein said device (10) has an unrotated position of repose and an infinite plurality of rotated positions relative to said unrotated position of repose.

8. The device (10) of claim 7, wherein
said unrotated position of repose aligns said device (10) with the gap between two adjacent vertebral bodies prior to insertion and during insertion of said device (10) into the gap between the two adjacent vertebral bodies;
said device (10) being in a rotated position after said distal retention plate (12) has exited said gap on a distal side of said gap, but prior to said proximal retention plate (16) entering said gap;
said device (10), when in said rotated position, preventing distal-to-proximal or proximal-to-distal displacement of said retention plates (12, 16) into said gap; and
said distal retention plate (12) when in said rotated position cooperating with said proximal retention plate (16) to hold the two adjacent vertebral bodies in a stable relationship to one another.

9. The device (10) of claim 8, wherein said distal part (14b) of the interconnecting member (14) of said distal retention plate (12) is telescopically mounted to said distal part (14b) of the interconnecting member (14) of said proximal retention plate (16) so as to permit retraction of said distal part (14b) and proximal part (14c), whereby to enable said proximal retention plate (16) and said distal retention plate (12) to positively engage the two adjacent vertebral bodies.

10. The device (10) of claim 2, wherein said proximal inner surface of said distal retention plate (12) and said distal inner surface of said proximal retention plate (16) comprise a roughened surface.

11. The device (10) of claim 2, wherein
said proximal retention plate (16) comprises screw-receiving holes formed therein so as to enable screws to fasten the proximal retention plate to the two adjacent vertebral bodies.

12. The device (10) of claim 1 wherein
said distal part (14b) of the interconnecting member (14)of said distal retention plate (12) comprises a non-round transverse cross-section and said proximal part (14c) of the interconnecting member (14) of said proximal retention plate (16) comprises a non-round transverse cross-section, and said distal part (14b) and proximal part (14c) of the interconnecting member (14) are disposed in sliding, mating relation to one another;
said distal part (14b) and proximal part (14c) of the interconnecting member (14) having a length sufficient to enable said distal retention plate (12) and said proximal retention plate (16) to extend beyond the two adjacent vertebral bodies;
said device (10) having an unrotated position of repose and an infinite plurality of rotated positions relative to said position of repose;
said unrotated position of repose aligning said device (10) with the gap between two adjacent vertebral bodies prior to insertion and during insertion of said device into the gap between two adjacent vertebral bodies;
said device (10) being in a rotated position after said distal retention plate (12) has exited the gap between two adjacent vertebral bodies on a distal side of the gap, but prior to said proximal retention plate (16) entering the gap;
said device (10), when in said rotated position, preventing distal-to-proximal or proximal-to-distal displacement of said distal retention plate (12) and said proximal retention plate (16) into the gap;
said distal retention plate (12), when in said rotated position, cooperating with said proximal retention plate (16) to hold the two adjacent vertebral bodies in a stable relationship to one another;
whereby rotation of said proximal retention plate (16) effects conjoint rotation of said distal retention plate (12); and
whereby a combined length of said distal part (14b) of the interconnecting member (14) of said distal retention plate (12) and said proximal part (14c) of the interconnecting member (14) of said proximal retention plate (16) is reduced so as to enable said proximal retention plate (16) and distal retention plate (12) to positively engage the two adjacent vertebral bodies.

13. The device (10) of claim 12, wherein said distal part (14b) of the interconnecting member (14) of said distal retention plate (12) comprises a non-round lumen;
said proximal part (14c) of the interconnecting member (14) of said proximal retention plate (16) comprises a non-round transverse cross-section; and
said non-round transverse cross-section of said distal part (14b) of the interconnecting member (14) of said proximal retention plate (16) is slidably disposed within said non-round lumen of said proximal part (14c) of the interconnecting member (14) of said distal retention plate (12).

14. The device (10) of claim 12, wherein said proximal part (14c) of the interconnecting member (14) of said proximal retention plate (16) comprises a non-round lumen;
said distal part (14b) of the interconnecting member (14) of said distal retention plate (12) comprises a non-round transverse cross-section; and
said non-round transverse cross-section of said distal part (14b) of the interconnecting member (14) of said distal retention plate (12) is slidably disposed within said non-round lumen of said proximal part (14c) of the interconnecting member (14) of said proximal retention plate (16).

## Patentansprüche

1. Vorrichtung (10) zum Einsetzen in den Spalt zwischen zwei benachbarten Wirbelkörpern, umfassend:
ein längliches Verbindungselement (14);
eine distale Halteplatte (12), die eine distale Außenfläche und eine proximale Innenfläche umfasst, wobei die distale Halteplatte (12) einen distalen Teil (14b) des Verbindungselements (14) aufweist, der in senkrechter Beziehung dazu befestigt ist und sich proximal davon erstreckt;
eine proximale Halteplatte (16), die eine distale Innenfläche und eine proximale Außenfläche umfasst, wobei die proximale Halteplatte (16) einen proximalen Teil (14c) des Verbindungselements (14) aufweist, der in senkrechter Beziehung dazu befestigt ist und sich distal davon erstreckt;
wobei der distale Teil (14b) des Verbindungselements (14) der distalen Halteplatte (12) teleskopisch an dem proximalen Teil (14c) des Verbindungselements (14) der proximalen Halteplatte (16) angebracht ist, um das Verbindungselement (14) zum selektiven Bewegen der distalen Halteplatte (12) von der proximalen Halteplatte (16) weg oder zu ihr hin und zum selektiven Drehen der distalen Halteplatte (12) relativ zu der proximalen Halteplatte (16) und zum selektiven Drehen der proximalen Halteplatte (16) relativ zu der distalen Halteplatte (12) zu bilden;
wobei die Vorrichtung (10) zum Einsetzen in den Spalt zwischen zwei benachbarten Wirbelkörpern angepasst ist und das Verbindungselement eine maximale Länge aufweist, die ausreicht, um die proximale bis distale Ausdehnung des Spalts zu überbrücken und eine Positionierung der proximalen und distalen Halteplatten außerhalb des Spalts zu ermöglichen, und eine minimale Länge aufweist, die ausreicht, damit die distalen und proximalen Halteplatten die benachbarten Körper fest umklammern und zwischen ihnen einklemmen können.

2. Vorrichtung (10) nach Anspruch 1, wobei die distale Halteplatte (12) und die proximale Halteplatte (16) ausreichend groß sind, um einen Abstand zwischen den benachbarten Wirbelkörpern zu überbrücken, wenn sie nach dem Einsetzen gedreht werden.

3. Vorrichtung (10) nach Anspruch 2, wobei der distale Teil (14b) des Verbindungselements (14) der distalen Halteplatte (12) und der proximale Teil (14c) des Verbindungselements (14) der proximalen Halteplatte (16) teleskopartig und drehfest aneinander befestigt sind.

4. Vorrichtung (10) nach Anspruch 3, wobei der distale Teil (14b) des Verbindungselements (14) der distalen Halteplatte (12) und der proximale Teil (14c) des Verbindungselements (14) der proximalen Halteplatte (16) einen unrunden Querschnitt umfassen; und
der distale Teil (14b) des Verbindungselements (14) der distalen Halteplatte (12) einen unrunden Hohlraum umfasst, der teleskopisch mit einem unrunden Querschnitt des proximalen Teils (14c) des Verbindungselements (14) der proximalen Halteplatte (16) zusammenpasst;
wobei eine Drehung der proximalen Halteplatte (16) eine gemeinsame Drehung der distalen Halteplatte (12) bewirkt.

5. Vorrichtung (10) nach Anspruch 3, wobei der distale Teil (14b) des Verbindungselements (14) der distalen Halteplatte (12) und der proximale Teil (14c) des Verbindungselements (14) der proximalen Halteplatte (16) einen unrunden Querschnitt umfassen; und
der proximale Teil (14c) des Verbindungselements (14) der proximalen Halteplatte (16) eine Hülse umfasst, die einen unrunden Hohlraum aufweist, wobei die Hülse so konfiguriert ist, dass sie teleskopisch mit einem unrunden Querschnitt des distalen Teils (14b) des Verbindungselements (14) der distalen Halteplatte (12) zusammenpasst;
wobei eine Drehung der proximalen Halteplatte (16) eine gemeinsame Drehung der distalen Halteplatte (12) bewirkt.

6. Vorrichtung (10) nach Anspruch 2, wobei der distale Teil (14b) des Verbindungselements (14) der distalen Halteplatte (12) und der proximale Teil (14c) des Verbindungselements (14) der proximalen Halteplatte (16) eine Länge aufweisen, die ausreicht, um die proximale Halteplatte (16) und die distale Halteplatte (12) über die benachbarten Wirbelkörper hinausragen zu lassen.

7. Vorrichtung (10) nach Anspruch 2, wobei die Vorrichtung (10) eine ungedrehte Ruhestellung und eine unendliche Vielzahl von gedrehten Stellungen relativ zu der ungedrehten Ruhestellung aufweist.

8. Vorrichtung (10) nach Anspruch 7, wobei
die ungedrehte Ruhestellung die Vorrichtung (10) auf den Spalt zwischen zwei benachbarten Wirbelkörpern vor dem Einsetzen und während des Einsetzens der Vorrichtung (10) in den Spalt zwischen den beiden benachbarten Wirbelkörpern ausrichtet;
sich die Vorrichtung (10) in einer gedrehten Stellung befindet, nachdem die distale Halteplatte (12) aus dem Spalt an einer distalen Seite des Spalts ausgetreten ist, jedoch bevor die proximale Halteplatte (16) in den Spalt eintritt;
die Vorrichtung (10) in der gedrehten Stellung eine Verschiebung der Halteplatten (12, 16) von distal nach proximal oder von proximal nach distal in den Spalt verhindert; und
die distale Halteplatte (12) in der gedrehten Stellung mit der proximalen Halteplatte (16) zusammenwirkt, um die beiden benachbarten Wirbelkörper in einer stabilen Beziehung zueinander zu halten.

9. Vorrichtung (10) nach Anspruch 8, wobei der distale Teil (14b) des Verbindungselements (14) der distalen Halteplatte (12) teleskopisch an dem distalen Teil (14b) des Verbindungselements (14) der proximalen Halteplatte (16) angebracht ist, um ein Zurückziehen des distalen Teils (14b) und des proximalen Teils (14c) zu ermöglichen, wodurch die proximale Halteplatte (16) und die distale Halteplatte (12) in die Lage versetzt werden, die beiden benachbarten Wirbelkörper formschlüssig zu verbinden.

10. Vorrichtung (10) nach Anspruch 2, wobei die proximale Innenfläche der distalen Halteplatte (12) und die distale Innenfläche der proximalen Halteplatte (16) eine aufgeraute Oberfläche umfassen.

11. Vorrichtung (10) nach Anspruch 2, wobei
die proximale Halteplatte (16) Schraubenaufnahmelöcher umfasst, die darin ausgebildet sind, um die Befestigung der proximalen Halteplatte an den beiden benachbarten Wirbelkörpern durch Schrauben zu ermöglichen.

12. Vorrichtung (10) nach Anspruch 1, wobei
der distale Teil (14b) des Verbindungselements (14) der distalen Halteplatte (12) einen unrunden Querschnitt umfasst und der proximale Teil (14c) des Verbindungselements (14) der proximalen Halteplatte (16) einen unrunden Querschnitt umfasst, und der distale Teil (14b) und der proximale Teil (14c) des Verbindungselements (14) in gleitender, zusammenpassender Beziehung zueinander angeordnet sind;
der distale Teil (14b) und der proximale Teil (14c) des Verbindungselements (14) eine Länge aufweisen, die ausreicht, um die distale Halteplatte (12) und die proximale Halteplatte (16) über die beiden benachbarten Wirbelkörper hinausragen zu lassen;
die Vorrichtung (10) eine ungedrehte Ruhestellung und eine unendliche Vielzahl von gedrehten Stellungen relativ zu der Ruhestellung aufweist;
die ungedrehte Ruhestellung die Vorrichtung (10) auf den Spalt zwischen zwei benachbarten Wirbelkörpern vor dem Einsetzen und während des Einsetzens der Vorrichtung in den Spalt zwischen zwei benachbarten Wirbelkörpern ausrichtet;
sich die Vorrichtung (10) in einer gedrehten Stellung befindet, nachdem die distale Halteplatte (12) aus dem Spalt zwischen zwei benachbarten Wirbelkörpern auf einer distalen Seite des Spalts ausgetreten ist, jedoch bevor die proximale Halteplatte (16) in den Spalt eintritt;
die Vorrichtung (10), wenn sie sich in der gedrehten Stellung befindet, eine Verschiebung der distalen Halteplatte (12) und der proximalen Halteplatte (16) von distal nach proximal oder von proximal nach distal in den Spalt verhindert;
die distale Halteplatte (12) in der gedrehten Stellung mit der proximalen Halteplatte (16) zusammenwirkt, um die beiden benachbarten Wirbelkörper in einer stabilen Beziehung zueinander zu halten;
wobei eine Drehung der proximalen Halteplatte (16) eine gemeinsame Drehung der distalen Halteplatte (12) bewirkt; und
wodurch eine kombinierte Länge des distalen Teils (14b) des Verbindungselements (14) der distalen Halteplatte (12) und des proximalen Teils (14c) des Verbindungselements (14) der proximalen Halteplatte (16) reduziert wird, um die proximale Halteplatte (16) und die distale Halteplatte (12) in die Lage zu versetzen, die beiden benachbarten Wirbelkörper formschlüssig zu verbinden.

13. Vorrichtung (10) nach Anspruch 12, wobei der distale Teil (14b) des Verbindungselements (14) der distalen Halteplatte (12) einen unrunden Hohlraum umfasst;
der proximale Teil (14c) des Verbindungselements (14) der proximalen Halteplatte (16) einen unrunden Querschnitt umfasst; und
der unrunde Querschnitt des distalen Teils (14b) des Verbindungselements (14) der proximalen Halteplatte (16) gleitend innerhalb des unrunden Hohlraums des proximalen Teils (14c) des Verbindungselements (14) der distalen Halteplatte (12) angeordnet ist.

14. Vorrichtung (10) nach Anspruch 12, wobei der proximale Teil (14c) des Verbindungselements (14) der proximalen Halteplatte (16) einen unrunden Hohlraum umfasst;
der distale Teil (14b) des Verbindungselements (14) der distalen Halteplatte (12) einen unrunden Querschnitt umfasst; und
der unrunde Querschnitt des distalen Teils (14b) des Verbindungselements (14) der distalen Halteplatte (12) gleitend innerhalb des unrunden Hohlraums des proximalen Teils (14c) des Verbindungselements (14) der proximalen Halteplatte (16) angeordnet ist.

## Revendications

1. Dispositif (10) destiné à être inséré dans l'espace entre deux corps vertébraux adjacents, comprenant :
un élément d'interconnexion allongé (14)
une plaque de retenue distale (12) comprenant une surface extérieure distale et une surface intérieure proximale, ladite plaque de retenue distale (12) présentant une partie distale (14b) de l'élément d'interconnexion (14) fixée en relation normale avec celle-ci et s'étendant de manière proximale à partir de celle-ci ;
une plaque de retenue proximale (16) comprenant une surface intérieure distale et une surface extérieure proximale, ladite plaque de retenue proximale (16) présentant une partie proximale (14c) de l'élément d'interconnexion (14) fixée en relation normale avec celle-ci et s'étendant de manière distale à partir de celle-ci ;
ladite partie distale (14b) de l'élément d'interconnexion (14) de ladite plaque de retenue distale (12) étant montée de manière télescopique sur ladite partie proximale (14c) de l'élément d'interconnexion (14) de ladite plaque de retenue proximale (16) pour former l'élément d'interconnexion (14) pour éloigner ou rapprocher sélectivement ladite plaque de retenue distale (12) de ladite plaque de retenue proximale (16), et pour tourner sélectivement ladite plaque de retenue distale (12) par rapport à ladite plaque de retenue proximale (16) et pour tourner sélectivement ladite plaque de retenue proximale (16) par rapport à ladite plaque de retenue distale (12) ;
dans lequel ledit dispositif (10) est adapté pour être inséré dans l'espace entre deux corps vertébraux adjacents et l'élément d'interconnexion présente une longueur maximale suffisante pour couvrir l'étendue proximale à distale de l'espace et pour permettre aux plaques de retenue proximale et distale d'être positionnées à l'extérieur de l'espace, et une longueur minimale suffisante pour permettre aux plaques de retenue distale et proximale de serrer étroitement les corps adjacents en sandwich entre celles-ci.

2. Dispositif (10) selon la revendication 1, dans lequel ladite plaque de retenue distale (12) et ladite plaque de retenue proximale (16) sont suffisamment grandes pour couvrir une distance entre lesdits corps vertébraux adjacents lorsqu'elles sont tournées après insertion.

3. Dispositif (10) selon la revendication 2, dans lequel ladite partie distale (14b) de l'élément d'interconnexion (14) de ladite plaque de retenue distale (12) et ladite partie proximale (14c) de l'élément d'interconnexion (14) de ladite plaque de retenue proximale (16) sont fixées l'une à l'autre selon une relation télescopique et non rotative.

4. Dispositif (10) selon la revendication 3, dans lequel ladite partie distale (14b) de l'élément d'interconnexion (14) de ladite plaque de retenue distale (12) et ladite partie proximale (14c) de l'élément d'interconnexion (14) de ladite plaque de retenue proximale (16) comprennent une section transversale non circulaire ; et
ladite partie distale (14b) de l'élément d'interconnexion (14) de ladite plaque de retenue distale (12) comprend une lumière non circulaire qui s'apparie de manière télescopique avec une section transversale non circulaire de ladite partie proximale (14c) de l'élément d'interconnexion (14) de ladite plaque de retenue proximale (16) ;
selon lequel une rotation de ladite plaque de retenue proximale (16) provoque une rotation conjointe de ladite plaque de retenue distale (12).

5. Dispositif (10) selon la revendication 3, dans lequel ladite partie distale (14b) de l'élément d'interconnexion (14) de ladite plaque de retenue distale (12) et ladite partie proximale (14c) de l'élément d'interconnexion (14) de ladite plaque de retenue proximale (16) comprennent une section transversale non circulaire ; et
ladite partie proximale (14c) de l'élément d'interconnexion (14) de ladite plaque de retenue proximale (16) comprend un manchon présentant une lumière non circulaire, ledit manchon étant configuré pour s'apparier de manière télescopique avec une section transversale non circulaire de ladite partie distale (14b) de l'élément d'interconnexion (14) de ladite plaque de retenue distale (12) ;
selon lequel une rotation de ladite plaque de retenue proximale (16) provoque une rotation conjointe de ladite plaque de retenue distale (12).

6. Dispositif (10) selon la revendication 2, dans lequel ladite partie distale (14b) de l'élément d'interconnexion (14) de ladite plaque de retenue distale (12) et ladite partie proximale (14c) de l'élément d'interconnexion (14) de ladite plaque de retenue proximale (16) présentent une longueur suffisante pour permettre à ladite plaque de retenue proximale (16) et à ladite plaque de retenue distale (12) de s'étendre au-delà des corps vertébraux adjacents.

7. Dispositif (10) selon la revendication 2, dans lequel ledit dispositif (10) présente une position de repos non tournée et une pluralité infinie de positions tournées par rapport à ladite position de repos non tournée.

8. Dispositif (10) selon la revendication 7, dans lequel
ladite position de repos non tournée aligne ledit dispositif (10) avec l'espace entre deux corps vertébraux adjacents avant une insertion et pendant une insertion dudit dispositif (10) dans l'espace entre les deux corps vertébraux adjacents ;
ledit dispositif (10) étant dans une position tournée après que ladite plaque de retenue distale (12) est sortie dudit espace sur un côté distal dudit espace, mais avant que ladite plaque de retenue proximale (16) n'entre dans ledit espace ;
ledit dispositif (10), lorsqu'il est dans ladite position tournée, empêchant un déplacement distal à proximal ou proximal à distal desdites plaques de retenue (12, 16) dans ledit espace ; et
ladite plaque de retenue distale (12), lorsqu'elle est dans ladite position tournée, coopérant avec ladite plaque de retenue proximale (16) pour maintenir les deux corps vertébraux adjacents dans une relation stable l'un par rapport à l'autre.

9. Dispositif (10) selon la revendication 8, dans lequel ladite partie distale (14b) de l'élément d'interconnexion (14) de ladite plaque de retenue distale (12) est montée de manière télescopique sur ladite partie distale (14b) de l'élément d'interconnexion (14) de ladite plaque de retenue proximale (16) de manière à permettre une rétraction de ladite partie distale (14b) et de ladite partie proximale (14c), permettant ainsi à ladite plaque de retenue proximale (16) et à ladite plaque de retenue distale (12) de venir en prise positive les deux corps vertébraux adjacents.

10. Dispositif (10) selon la revendication 2, dans lequel ladite surface interne proximale de ladite plaque de retenue distale (12) et ladite surface interne distale de ladite plaque de retenue proximale (16) comprennent une surface rendue rugueuse.

11. Dispositif (10) selon la revendication 2, dans lequel
ladite plaque de retenue proximale (16) comprend des trous de réception de vis formés dans celle-ci de manière à permettre à des vis de fixer la plaque de retenue proximale aux deux corps vertébraux adjacents.

12. Dispositif (10) selon la revendication 1, dans lequel
ladite partie distale (14b) de l'élément d'interconnexion (14) de ladite plaque de retenue distale (12) comprend une section transversale non circulaire et ladite partie proximale (14c) de l'élément d'interconnexion (14) de ladite plaque de retenue proximale (16) comprend une section transversale non circulaire, et lesdites partie distale (14b) et partie proximale (14c) de l'élément d'interconnexion (14) sont disposées en relation d'appariement coulissante l'une par rapport à l'autre ;
lesdites partie distale (14b) et partie proximale (14c) de l'élément d'interconnexion (14) présentant une longueur suffisante pour permettre à ladite plaque de retenue distale (12) et à ladite plaque de retenue proximale (16) de s'étendre au-delà des deux corps vertébraux adjacents ;
ledit dispositif (10) présentant une position de repos non tournée et une pluralité infinie de positions tournées par rapport à ladite position de repos ;
ladite position de repos non tournée alignant ledit dispositif (10) avec l'espace entre deux corps vertébraux adjacents avant une insertion et pendant une insertion dudit dispositif dans l'espace entre deux corps vertébraux adjacents ;
ledit dispositif (10) étant dans une position tournée après que ladite plaque de retenue distale (12) est sortie de l'espace entre deux corps vertébraux adjacents sur un côté distal de l'espace, mais avant que ladite plaque de retenue proximale (16) n'entre dans l'espace ;
ledit dispositif (10), lorsqu'il est dans ladite position tournée, empêchant un déplacement distal à proximal ou proximal à distal de ladite plaque de retenue distale (12) et de ladite plaque de retenue proximale (16) dans l'espace ;
ladite plaque de retenue distale (12), lorsqu'elle est dans ladite position tournée, coopérant avec ladite plaque de retenue proximale (16) pour maintenir les deux corps vertébraux adjacents dans une relation stable l'un par rapport à l'autre ;
selon lequel une rotation de ladite plaque de retenue proximale (16) provoque une rotation conjointe de ladite plaque de retenue distale (12) ; et
selon lequel une longueur combinée de ladite partie distale (14b) de l'élément d'interconnexion (14) de ladite plaque de retenue distale (12) et de ladite partie proximale (14c) de l'élément d'interconnexion (14) de ladite plaque de retenue proximale (16) est réduite de manière à permettre à ladite plaque de retenue proximale (16) et à ladite plaque de retenue distale (12) de venir en prise positive les deux corps vertébraux adjacents.

13. Dispositif (10) selon la revendication 12, dans lequel ladite partie distale (14b) de l'élément d'interconnexion (14) de ladite plaque de retenue distale (12) comprend une lumière non circulaire ;
ladite partie proximale (14c) de l'élément d'interconnexion (14) de ladite plaque de retenue proximale (16) comprend une section transversale non circulaire ; et
ladite section transversale non circulaire de ladite partie distale (14b) de l'élément d'interconnexion (14) de ladite plaque de retenue proximale (16) est disposée de manière coulissante à l'intérieur de ladite lumière non circulaire de ladite partie proximale (14c) de l'élément d'interconnexion (14) de ladite plaque de retenue distale (12).

14. Dispositif (10) selon la revendication 12, dans lequel ladite partie proximale (14c) de l'élément d'interconnexion (14) de ladite plaque de retenue proximale (16) comprend une lumière non circulaire ;
ladite partie distale (14b) de l'élément d'interconnexion (14) de ladite plaque de retenue distale (12) comprend une section transversale non circulaire ; et
ladite section transversale non circulaire de ladite partie distale (14b) de l'élément d'interconnexion (14) de ladite plaque de retenue distale (12) est disposée de manière coulissante à l'intérieur de ladite lumière non circulaire de ladite partie proximale (14c) de l'élément d'interconnexion (14) de ladite plaque de retenue proximale (16).
